Europäisches Patentamt

European Patent Office

Office européen des brevets

(11) Numéro de publication: **0 357 476**
**A1**

(12) **DEMANDE DE BREVET EUROPEEN**

(21) Numéro de dépôt: 89402039.5

(22) Date de dépôt: 18.07.89

(51) Int. Cl.⁵: **C 12 P 19/12**
C 07 H 13/04, A 23 L 1/236

(30) Priorité: 20.07.88 FR 8809785

(43) Date de publication de la demande:
07.03.90 Bulletin 90/10

(84) Etats contractants désignés:
BE CH DE ES FR GB IT LI NL

(71) Demandeur: **BEGHIN-SAY SOCIETE ANONYME**
**F-59239 Thumeries (FR)**

(72) Inventeur: **Guibert, Alain**
**11, rue Emile Zola**
**F-31520 Ramonville St Agne (FR)**

**Mentech, Julio**
**1d, rue Phelypeaux**
**F-69100 Villeurbanne (FR)**

(74) Mandataire: **Dorland, Anne-Marie**
**BEGHIN-SAY 54, avenue Hache**
**F-75008 Paris (FR)**

(54) Nouveau procédé d'obtention de dérivés du saccharose modifiés en position 4'.

(57) Nouveau procédé d'obtention de dérivés du saccharose modifiés en position 4' consistant à

a) effectuer l'estérification de toutes les fonctions hydroxyle du produit de départ saccharose ou dérivé substitué du saccharose pour donner un perester dudit produit de départ ;

b) effectuer une hydrolyse partielle, à l'aide d'une enzyme lipolytique dudit perester de l'étape a) conduisant à un ester de saccharose ayant une fonction hydroxyle libre en position 4' ;

c) effectuer toute réaction appropriée sur ladite fonction hydroxyle libre en position 4' du produit de l'étape b) pour obtenir le dérivé du saccharose modifié en position 4' souhaité.

L'étape b) de ce procédé conduit, de façon simple et avec une régiosélectivité remarquable à des monoalcools dérivés du saccharose ayant une fonction hydroxyle libre en position 4', intermédiaires de synthèse de dérivés du saccharose modifiés en position 4'.

Les dérivés du saccharose modifiés en position 4' ont des propriétés intéressantes, comme par exemple un pouvoir édulcorant très élevé dans le cas où l'hyroxyle en position 4' est remplacé par un atome d'halogène.

**Description**

## NOUVEAU PROCEDE D'OBTENTION DE DERIVES DU SACCHAROSE MODIFIES EN POSITION 4'

La présente invention concerne un nouveau procédé d'obtention de dérivés du saccharose modifiés en position 4' et plus particulièrement un procédé permettant d'obtenir sélectivement des intermédiaires de synthèse monoalcools dérivés du saccharose dont la fonction alcool est en position 4' (les numéros avec prime correspondent au noyau fructose du saccharose, les numéros sans prime correspondent au noyau glucose du saccharose).

Le remplacement dans le saccharose ou ses dérivés substitués du groupement hydroxyle en position 4' peut conduire à des modifications importantes des propriétés de la molécule du composé de départ, comme par exemple l'augmentation remarquable du pouvoir édulcorant lorsque l'hydroxyle en position 4' est remplacé par un atome d'halogène dans la série des chlorosaccharoses décrite dans l'EP-A-0073093.

Des méthodes ont été proposées pour attaquer directement la position 4' du saccharose, comme par exemple l'attaque par du chlorure de sulfuryle (J.C.S. Perkin I, 1973, 1524), mais une telle attaque n'est pas sélective et on obtient des mélanges complexes de produits.

Il existe dans la littérature des méthodes d'accès à la position 4', comme par exemple celle proposée par R. KHAN et al (Carbohydr. Res. 1987, 162, 199) consistant à protéger convenablement le saccharose par acétonation et acétylation partielle, à époxyder ensuite les positions 3' et 4', ouvrir l'époxyde ainsi formé par un réactif convenable et à libérer les positions initialement protégées. Cette méthode présente l'inconvénient de comporter de nombreuses étapes.

Le même type d'approche est suivi par C.K. LEE (Carbohydr. Res. 1987, 162, 53).

La demanderesse a découvert de façon surprenante qu'il était possible de réaliser, au moyen d'une hydrolyse enzymatique appropriée, une désestérification remarquablement régiosélective de peresters du saccharose ou d'un dérivé substitué du saccharose. Par "perester", il faut entendre dans la présente description, le produit où toutes les fonctions hydroxyle du saccharose ou du dérivé substitué du saccharose de départ ont été estérifiées ; dans le cas du saccharose ledit perester est un octaester.

Le procédé d'obtention de dérivés du saccharose modifiés en position 4' de la présente invention consiste donc à :

    a) effectuer l'estérification de toutes les fonctions hydroxyle du produit de départ saccharose ou dérivé substitué du saccharose pour donner un perester dudit produit de départ ;

    b) effectuer une hydrolyse partielle dudit perester de l'étape a) conduisant à un ester de saccharose ayant une fonction hydroxyle libre en position 4' ;

    c) effectuer toute réaction appropriée sur ladite fonction hydroxyle libre en position 4' du produit de l'étape b) pour obtenir le dérivé du saccharose modifié en position 4' souhaité ; ce procédé étant caractérisé par le fait que ladite hydrolyse partielle de l'étape b) est effectuée à l'aide d'une enzyme lipolytique.

L'estérification de l'étape a) ci-dessus est effectuée de façon connue en soi, à l'aide d'un chlorure d'acide, d'un anhydride d'acide, ou par toute autre méthode bien connue de l'homme de l'art, pour obtenir un perester dont les groupements acyle, identiques ou différents, peuvent avoir de 1 à 30 atomes de carbone. Lesdits groupements acyle seront commodément chacun le groupement acétyle.

L'hydrolyse d'esters du saccharose a été réalisée antérieurement par voie chimique (voir par exemple Collect. Czeckoslovak Chem. Commun. 1986, 51, 1476) mais une telle hydrolyse conduit à des mélanges de monoalcools sans régiosélectivité, contrairement à ce que l'on obtient par la voie enzymatique de l'invention.

L'hydrolyse partielle de l'étape b) est effectuée selon la présente invention à l'aide d'une enzyme lipolytique, c'est-à-dire une enzyme appartenant à la classe des lipases.

Parmi les lipases utilisables dans la présente invention, on peut citer de façon non limitative les lipases pancréatiques, les lipases obtenues à partir de Candida telles les Candida cylindracea, de germe de blé, d'Aspergillus telles les Aspergillus niger, de Pseudomonas telles les Pseudomonas fluorescens, de Mucor, d'Enterobacterium, de Chromobacterium, de Geotrichum, de Penicillum, de Rhizopus, etc...

L'enzyme peut être immobilisée ou non.

L'hydrolyse partielle selon l'invention est effectuée en milieu biphasique eau/solvant organique généralement dans le rapport eau/solvant organique d'environ 9/1, ce qui permet de recycler la phase aqueuse contenant l'enzyme et de récupérer les produits obtenus par simple séparation de la phase organique. On choisit le solvant organique de façon à obtenir la meilleure activité possible de l'enzyme ; on peut citer à titre d'exemples non limitatifs de solvants organiques utilisables dans la présente invention : le toluène, le xylène, le benzène, l'hexane, le chloroforme, le dichlorométhane, l'éther de pétrole, le glyme, le diglyme, l'éther éthylique, l'éther propylique, l'éther butylique, etc... La température est choisie en fonction de l'activité optimale de l'enzyme et se situe en général entre environ 15 et 70°C. Le pH est maintenu constant, par exemple au moyen d'un pH-STAT automatique, généralement dans l'intervalle compris entre 6 et 9. La réaction peut également être favorisée par l'addition de sels minéraux tels $CaCl_2$, $MgCl_2$, $MgSO_4$, $SrCl_2$, etc...

L'hydrolyse enzymatique sera poursuivie pendant une durée suffisante pour obtenir l'hydrolyse d'une fonction hydroxyle dudit perester, et obtenir, selon la présente invention, un ester de saccharose ayant une fonction hydroxyle libre en position 4'. L'hydrolyse peut être poursuivie et, selon les conditions mises en oeuvre, on obtient un ester de saccharose pouvant avoir plusieurs fonctions hydroxyle libres, dont une en position 4'.

L'homme de l'art déterminera aisément, au moyen de techniques analytiques bien connues, le temps au bout duquel la réaction doit être stoppée pour n'obtenir que le monoalcool dont la fonction hydroxyle se trouve, selon l'invention, en position 4'.

Une fois l'hydrolyse terminée, la phase organique contenant le produit souhaité peut être évaporée et traitée de façon classique (cristallisation, extraction, chromatographie) afin d'isoler l'intermédiaire recherché, ou bien peut être traitée directement pour obtenir, par des réactions connues, des dérivés du saccharose modifiés en position 4'.

L'exemple ci-après illustre l'invention de façon non limitative.

## Exemple

### Préparation du 1',2,3,3',4,6,6'-hepta-O-acétyl-β- D-fructofuranosyl-α- D-glucopyranose (I)

Une solution d'octaacétate de saccharose (678 mg, 1 mmol) dans 1 ml de toluène est mise en contact avec 9 ml d'une solution de 100 mg de lipase de Candida cylindracea (Lipase AY AMANO 30) dans un tampon phosphate (0,1 M, pH 7). Le mélange est fortement agité à 45°C et le pH maintenu à une valeur constante de pH égale à 7 par addition automatique d'une solution de soude $10^{-3}$ M. Après 48 heures de réaction les deux phases sont séparées. La phase organique est lavée à l'eau et la phase aqueuse au toluène. Les fractions organiques sont rassemblées ainsi que les différentes fractions aqueuses. La phase aqueuse est mise en contact avec une nouvelle solution d'octaacétate de saccharose dans le toluène et recyclée ainsi plusieurs fois. La phase organique est séchée, évaporée et l'heptaacétate de saccharose (1) purifié sur colonne de silice avec un éluant composé d'acétate d'éthyle : hexane 80 : 20. Le rendement en produit I est de 40 % (les 60 % restants représentent pratiquement l'octaacétate de saccharose de départ non-réagi qui peut être recyclé). L' heptaacétate de saccharose obtenu peut être mis en réaction sans séparation préalable.

Microanalyse expérimentale calculée pour $C_{26}H_{36}O_{18}$ : C 49,05 ; H 5,70
C 48,99 ; H 5,40
$[\alpha]_D^{20}$ + 53,8 (C 0,5, CHCL$_3$)
r.m.n. $^1$H (C$_6$D$_6$, 300 MHz) 5,84(d,1H,H-1,J$_{1,2}$=3,8) 5,81(dd,1H,H-3,J$_{3,4}$=9,8) 5,52(d,1H,H-3',J$_{3',4'}$=7,5) 5,30(dd,1H,H-4,J$_{4,5}$=9,8) 5,07(dd, 1H,H-2,J$_{2,3}$=10,2) 4,48(m,1H,H-5)
4,40-4,26(m,7H,H-6a,H-6b,-H-6'a,H-6'b,H-1'a,H-1'b,-H-4') 4,15(m,1H,H-5').

## Revendications

1) Procédé d'obtention de dérivés du saccharose modifiés en position 4' consistant à effectuer les étapes suivantes :
a) estérification de toutes les fonctions hydroxyle du produit de départ choisi parmi le saccharose et les dérivés substitués du saccharose, conduisant à un perester dudit produit de départ ;
b) hydrolyse partielle dudit perester obtenu dans l' étape a), par laquelle on obtient un ester de saccharose ayant une fonction hydroxyle libre en position 4' ;
c) Mise en réaction appropriée dudit ester de saccharose ayant une fonction hydroxyle libre en position 4' provenant de l'étape b) conduisant à un dérivé du saccharose modifié en position 4' ;
caractérisé par le fait que ladite hydrolyse partielle de l'étape b) est effectuée à l'aide d'une enzyme lipolytique.

2. Procédé selon la revendication 1, caractérisé en ce que ladite enzyme lipolytique est choisie parmi les lipases pancréatiques, les lipases obtenues à partir de Candida, telles les Candida cylindracea, de germe de blé, d'Aspergillus, telles les Aspergillus niger, de Pseudomonas telles les Pseudomonas fluorescens, de Mucor, d'Enterobacterium, de Chromobacterium, de Geotrichum, de Penicillium, de Rhizopus, etc. ..

3. Procédé selon la revendication 2, dans lequel ladite enzyme lypolitique est une lipase de Candida cylindracea.

4. Procédé selon l'une des revendications 1 à 3, dans lequel ladite hydrolyse partielle est effectuée en milieu biphasique eau/solvant organique.

5. Procédé selon la revendication 4, dans lequel ledit solvant organique est choisi parmi le toluène, le xylène, le benzène, l'hexane, le chloroforme, le dichlorométhane, l'éther de pétrole, le glyme, le diglyme, l'éther éthylique, l'éther propylique et l'éther butylique.

6. Procédé selon l'une des revendications 1 à 5 dans lequel ledit produit de départ est le saccharose.

7. Procédé selon l'une des revendications 1 à 6 dans lequel les groupements acyle dudit perester formé dans l'étape a) sont identiques ou différents et choisis parmi les groupements alkyle ayant de 1 à 30 atomes de carbone.

8. Procédé selon la revendication 7 dans lequel lesdits groupements acyle sont chacun le groupement acétyle.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,Y | CHEMICAL ABSTRACTS, vol. 109, no. 17, 24 octobre 1988, page 599, résumé no. 148020w, Columbus, Ohio; & JP-A-63 112 993 (DAIICHI KOGYO SEIYAKU CO. LTD) 18-05-1988 * Résumé; en entier * | 1-8 | C 12 P  19/12 C 07 H  13/04 A 23 L   1/236 |
| Y | PATENT ABSTRACTS OF JAPAN, vol. 6, no. 35 (C-93)[913], 3 mars 1982, page 6 C 93; & JP-A-56 151 496 (MITSUI TOATSU KAGAKU K.K.) 24-11-1981 * Résumé; en entier * | 1-8 | |
| D,A | COLLECTION OF CZECHOLOVAK CHEMICAL COMMUNICATIONS, vol. 7, no. 51, 1986, pages 1476-1486; K. CAPEK et al.: "Partially acetylated sucrose. Structures of hepta-O-acetylsucroses formed by deacetylation of octa-O-acetylsucrose" | 1-8 | |
| Y | CAN. JOURNAL OF BIOCHEMISTRY, vol. 47, no. 3, 1969, pages 353-359; A.L. FINK et al.: "The enzymic deacylation of esterified mono- and di-saccharides. IV. The products of esterase-catalyzed deacetylations" * Page 358, tableau V * | 1-8 | **DOMAINES TECHNIQUES RECHERCHES (Int. Cl.5)**<br><br>C 07 H C 12 P |

Le présent rapport a été établi pour toutes les revendications

| Lieu de la recherche | Date d'achèvement de la recherche | Examinateur |
|---|---|---|
| LA HAYE | 03-11-1989 | LENSEN H.W.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P0402)